# EUROPÄISCHE PATENTSCHRIFT

(11) **EP 2 775 955 B1**
(45) Veröffentlichungstag und Bekanntmachung des Hinweises auf die Patenterteilung: **03.10.2018**
(21) Anmeldenummer: 12788440.1
(22) Anmeldetag: 09.11.2012
(51) Int. Cl.: A61C 13/20, A61C 13/00, A61C 13/01

(54) **FRÄSBLOCK, VERFAHREN ZUR HERSTELLUNG VON TEIL- ODER TOTALPROTHESEN SOWIE FRÄSBLOCKSYSTEM**
MILLING BLOCK, METHOD FOR PRODUCING PARTIAL OR TOTAL PROSTHESES AND MILLING BLOCK SYSTEM
BLOC À FRAISER, PROCÉDÉ DE FABRICATION DE PROTHÈSES PARTIELLES OU COMPLÈTES ET SYSTÈME DE BLOC À FRAISER

(30) Priorität: 11.11.2011 DE 102011118320
(43) Veröffentlichungstag der Anmeldung: 17.09.2014
(73) Patentinhaber: Merz Dental GmbH, 24321 Lütjenburg (DE)
(72) Erfinder: KLINGENBURG, Fiedhelm, 24321 Lütjenburg (DE); FRÖLICH, Carola, 89073 Ulm (DE)
(74) Vertreter: Kunz, Herbert
(86) Internationale Anmeldenummer: PCT/EP2012/004665
(87) Internationale Veröffentlichungsnummer: WO 2013/068124

(56) Entgegenhaltungen:
- WO-A1-2010/057584
- DE-A1- 4 025 728

## Beschreibung

Die Erfindung betrifft einen Fräsblock, ein Verfahren zur Herstellung von Teil- oder Totalprothesen, sowie ein komplettes Fräsblocksystem bestehend aus einem ersten Fräsblock für das Obergebiss und einem zweiten Fräsblock für das Untergebiss und ein Verfahren zur Bereitstellung einer Teil- oder Totalprothese.

Aus der EP 0 501 983 B1 ist bereits ein Verfahren zur Herstellung einer Totalprothese mit einer Ober- und/oder Unterkieferbasis mit Hilfe einer Fräseinrichtung bekannt, wobei die jeweilige Basis aus einem Kunststoffblock ausgefräst wird und die Kontur oder Topographie kennzeichnenden Daten der Mundhöhle verwendet werden. Dies darin beschriebene Verfahren verwendet ferner nach Erstellen der angepassten Basis an die Mundhöhle den weiteren Verfahrensschritt, notwendige Zähne in Wachs auf die Ober- und/oder Unterkieferbasis aufzustellen, wobei die Fertigstellung der Prothese durch Verbinden der Zähne mit der Basis mit Hilfe eines Kaltpolymerisates durchgeführt wird. Daran anschließend erfolgt das sogenannte Reokkludieren, damit dem jeweiligen Patient entsprechend seiner Kaubewegungen ein optimaler Biss vermittelt wird.

Dieses bekannte Verfahren beinhaltet somit den Nachteil, dass nach Fertigstellung der Ober- und/oder Unterkieferbasis ein individuelles Anpassen an den Patienten durch entsprechendes Aufstellen der Zähne in Wachs erforderlich wird, was wiederum den gesamten zahntechnischen und zahnärztlichen Behandlungsablauf zeit- und kostenintensiv macht.

Ferner wird in diesem Zusammenhang auf die DE4025728 A1 verwiesen. Aufgabe der folgenden Erfindung ist es somit, einen Fräsblock und ein Verfahren zur Herstellung von Teil- oder Totalprothesen sowie ein komplettes Fräsblocksystem bereitzustellen, welche die Nachteile des Standes der Technik vermeiden. Ferner ist es Aufgabe der folgenden Erfindung, die Arbeitsschritte zur individuellen Anpassung an den Patienten zu verringern.

Erfindungsgemäß wird zur Lösung der Aufgaben vorgeschlagen, dass ein Fräsblock zur Herstellung von Teil- oder Totalprothesen bereitgestellt wird, bei dem entsprechend der Kieferform eine zu bearbeitende Prothesenbasis mit künstlich geformten Zähnen eines vollständigen Gebisses vorgesehen ist. In diesem Sinne ist auch das entsprechende Fräsblocksystem hinsichtlich Obergebiss und Untergebiss unabhängig von der Befestigung im Mund ausgestattet.

Mit dieser anmeldungsgemäßen Maßnahme wird erreicht, dass lediglich die Prothesenbasis gefräst bzw. geformt werden muss, und bei korrekter Fräsung entsprechend vorliegender CAD/CAM-Daten der Mundhöhle des Patienten aufgrund bereits auf der Prothesenbasis verankerter künstlich geformter Zähne keine weitere Anpassung erfolgen muss.

Durch entsprechend optimierte Einpassung und Justierung der Prothesenbasis in die Mundhöhle wird somit nur ein individueller Anpassungsschritt erforderlich sein, um den Patienten optimal zu versorgen.

Aufgrund des anmeldungsgemäßen Vorhandenseins von einem Fräsblock bzw. Fräsblocksystem, bei dem eine entsprechende Kieferform zur bearbeitenden Prothesenbasis mit künstlich geformten Zähnen vorliegt, muss weder eine separate Bissnahme für eine Kieferrelationsbestimmung , noch eine zeit- und kostenintensive Anprobe vorgenommen werden.

Mit der anmeldungsgemäßen Maßnahme ist somit ein nachträgliches Einsetzen in einem Artikulator zur Bestimmung der Positionierung der Zähne nicht erforderlich, da die künstlich geformten Zähne bereits entsprechend gegebener Grunddaten für die Okklusionsstellung vorbereitet sind.

Mit einem Verfahren zur Bereitstellung einer Teil- oder Totalprothese ist es somit möglich, dass aufgrund des Vorhandenseins von in Okklusionsstellung vorliegenden Zahnober- und unterreihen in Gebißform mindestens drei unterschiedliche Fräsblocksysteme mit unterschiedlichen Größenverhältnissen in der Gebißform und Größe der Zähne bereitgestellt werden können, um nahezu alle Patienten versorgen zu können. Mit dieser Maßnahme wird ein zeit- und kosteneffizientes Herstellungsverfahren erzielt und dem Zahnarzt und/oder dem Zahntechniker in allen Ländern eine vollständige Versorgung der Bevölkerung ermöglicht.

Im Unterschied zu den bekannten Dentalprothesen bzw. deren Herstellungsverfahren richtet sich der erfindungsgemäße Fräsblock auf eine einstückige Anordnung mit einem ersten Bereich, welcher eine entsprechend der Kieferform bearbeitbare Prothesenbasis aufweist und einem zweiten Bereich mit einer vorbestimmten, bzw. standardisierten Anordnung künstlich geformter Zähne oder Zahnreihen für ein Ober-bzw. Unterkiefergebiss. Damit wird als Vorstufe zur Dentalprothese ein Fräsblock bereitgestellt, der besonders kosten- und zeitsparend lediglich in seinem ersten Bereich, nämlich der Prothesenbasis, zur Anpassung an die Kieferform bearbeitbar, d.h. fräsbar ist. Das erfindungsgemäße Fräsblocksystem umfasst zumindest einen ersten Fräsblock für das Obergebiss und einen zweiten Fräsblock für das Untergebiss, d.h. die Vorstufen einer Teil- oder Totalprothese, welche im jeweils zweiten Bereich des ersten und/oder zweiten Fräsblocks eine vorbestimmte bzw. standardisierte und nach gängigen Okklusionsprinzipien aufgestellte Anordnung künstlich geformter Zähne oder Zahnreihen für das Ober-bzw. Unterkiefergebiss aufweist.

Eine aus dem Stand der Technik bekannte Mehrzahl von Vorformlingen mit verschiedenen Farben, Größen- und Formenkombinationen für den Schleimhautbereich und Zahnbereich zur Herstellung von Zahnersatz wird mit vorliegendem Erfindungsgegenstand durch die vorbestimmte bzw. standardisierte Anordnung künstlich geformter, einpolymerisierter Zähne oder Zahnreihen für das Ober-bzw. Unterkiefergebiss erheblich vereinfacht. Vorteilhaft ist anmeldungsgemäß, dass lediglich nur die jeweilige, dem Schleimhautbereich zugeordnete Prothesenbasis zu fräsen ist.

Darüber hinaus handelt es sich bei der vorbestimmten bzw. standardisierten und nach gängigen Okklusionsprinzipien aufgestellten Anordnung künstlich geformter Zähne oder Zahnreihen für das Ober-bzw. Unterkiefergebiss um nicht-individualisierte, vorgefertigte Zahnersatzteile, die keiner weiteren Nachbearbeitung bedürfen.

Die vorbestimmte bzw. standardisierte und nach gängigen Okklusionsprinzipien aufgestellten Anordnung künstlich geformter Zähne oder Zahnreihen für das Ober-bzw. Unterkiefergebiss erfolgt nach einer Auswahl aus vorgefertigten Zahneratzteilen, d.h. künstlich geformter Zähne oder Zahnreihen nach vorgegebenen und bekannten Aufstellsystemen.

Weitere vorteilhafte Weiterbildungen der Erfindung sind Gegenstand der Unteransprüche.

Sind darüber hinaus die künstlich geformten Zähne und die Prothesenbasis einstückig ausgebildet, so kann bei der Herstellung des Fräsblockes nahezu eine Guss- bzw. Pressform verwendet werden, die somit auch jeweils nur ein Material für den Fräsblock verwendet, welches vorteilhaft sowohl ein geringeres Allergiepotential als auch eine höhere Verbundfestigkeit aufweist.

Ist vorteilhafterweise der Fräsblock zweiteilig ausgestaltet, so ist die Möglichkeit gegeben, dass der erste Bereich, der entsprechend der Kieferform bearbeitet wird, standardisiert hergestellt werden kann und mit unterschiedlichen, ebenfalls schon auf einem zweiten Bereich aufgestellten Zähnen, welche unterschiedliche Größen und Gegebenheiten aufweisen, zusammengefügt werden kann. Mit der Weiterbildung, dass die Prothesenbasis flexibel ausgestaltet wird, kann einer vereinfachten Einpassung in die Mundhöhle bzw. Anpassung an die Kieferform des jeweiligen Patienten Rechnung getragen werden.

Als besonders vorteilhafte Materialien für den Fräsblock bzw. für das Fräsblocksystem, d. h. Prothesenbasis und/oder Zähne, bieten sich u. a. sogenannte PMMA-Kunststoffe, ebenso als Thermoplaste, organisch als auch anorganisch gefüllte Kunststoffe und auch allergiearme Kunststoffe an. Zusätzlich ist es ebenfalls vorteilhaft, jedwede Form von Keramiken, insbesondere Glaskeramiken, leuzitverstärkte Keramik, aluminiumverstärkte Keramik, Oxidkeramik, Zirkonoxidkeramik, Infiltrationskeramik, Feldspatkeramiken, Lithium-Disilikat-Keramik, zu verwenden. Denkbar ist es ebenfalls, Materialien zu verwenden, die gesintert werden, die jedoch noch einen weiteren Fertigungsschritt benötigen. Verwendbar ist der Fräsblock bzw. das Fräsblocksystem auch bei kombiniertem Zahnersatz.

Sind die Zähne entsprechend einer Okklusionsstellung bereits ausgerichtet, wird somit nur durch entsprechende Fräsung der Prothesenbasis die vollständige Einpassung in die Mundhöhle des Patienten vorgenommen. Der Patient kann damit sicher gehen, dass bei entsprechender Anpassung in die Mundhöhle bzw. an den Ober- bzw. Unterkiefer eine optimale Okklusionsstellung bereitsteht und somit vorteilhaft eine hinsichtlich des Bissverhaltens uneingeschränkt funktionsfähige Totalprothese vorliegt. Aufgrund des anmeldungsgemäßen Fräsblockes bzw. Fräsblocksystems und dem entsprechenden Verfahren zur Herstellung von Teil- oder Totalprothesen ist es beispielsweise möglich, mindestens drei unterschiedliche Größen bereitzustellen, mit denen nahezu für jeden Patienten entsprechend seiner Gesichtsform und Größe der Mundhöhle eine vollständige Totalprothese bereitgestellt werden kann. Aufgrund der Einfachheit der Anpassung der Totalprothese sind somit die Arbeitsschritte zur Anpassung in die Mundhöhle erheblich reduziert und damit auch kostengünstiger. Je nach Erfahrungswerten kann bei bestimmten Bevölkerungsgruppen das Fräsblocksystem auch nur zwei Arten oder mehr beinhalten.

Hinsichtlich der Vorbereitung und Aufstellung der künstlich geformten Zähne sind bei dem anmeldungsgemäßen Fräsblock, Fräsblocksystem und dem entsprechenden Verfahren alle hierzu möglichen, bislang bekannten Aufstellsysteme einsetzbar, wie z. B. die sogenannten Uraufstellsysteme gemäß Gysi, Gerber und Schreinemaker. An die Aufstelltechniken von Gysi und Gerber anlehnende Aufstelltechniken gemäß APF und APF NT und TiF sind ebenfalls vorteilhaft einsetzbar. Gemäß Staub Cranial wird eine mathematisch berechnende Aufstelltechnik verwendet, die aufgrund von erstellten Messungen die ursprüngliche Position der Zähne bestimmt und dort entsprechend wieder positioniert.

Der anmeldungsgemäße Fräsblock bzw. das Fräsblocksystem ist insbesondere auch bei der Herstellung von Teilprothesen einsetzbar, insbesondere, wenn zumindest gegenüberliegende Quadranten des Ober- bzw. Untergebisses eingesetzt werden können. Aufgrund der für die Zähne in Okklusionsstellung vorbereiteten Aufstellung ist somit auch nach Einsetzen und Einpassen in den entsprechenden Kieferbereich eine Nachbearbeitung der Zähne praktisch nicht notwendig.

Eine besonders vorteilhafte Herstellungstechnik für den Fräsblock bzw. für das Fräsblocksystem ist die Möglichkeit, die ausgewählten Zähne in der Okklusionsstellung in dem ersten Bereich der Prothesenbasis vorpolymerisiert einzusetzen und dann erst bei dem Zusammenfügen mit dem zweiten Bereich der Prothesenbasis die Endpolymerisation vorzunehmen. Auf diese Weise wird erreicht, dass die jeweilige Ober- und Unterkieferprothese einstückig und form- und kraftschlüssig präpariert und hergestellt wird und somit ein geringeres Allergiepotential sowie eine höhere Verbundfestigkeit aufweist.

Weitergehende Ausbildungen der vorliegenden Erfindung sind Gegenstand der weiteren Unteransprüche.

Mit dem anmeldungsgemäßen Fräsblock bzw. Fräsblocksystem wird ermöglicht, dass eine Fixierung bzw. Kennzeichnung jeglicher Art für eine Nulllageübergabe bzw. Nullpunktsübergabe zur maschinellen CAD/CAM Bearbeitung vorgenommen werden kann, Dies ermöglicht eine enorme Zeitersparnis, insbesondere durch Wegfall des Abtastens, zur Erreichung der Lagepositionierung und verhindert Fehlbearbeitungen in einem CAD/CAM System

Der anmeldungsgemäße Fräsblock sowie das Fräsblocksystem und das Verfahren zur Herstellung von Teil- oder Totalprothesen werden anhand der Zeichnungen nachstehend beispielhaft erläutert.

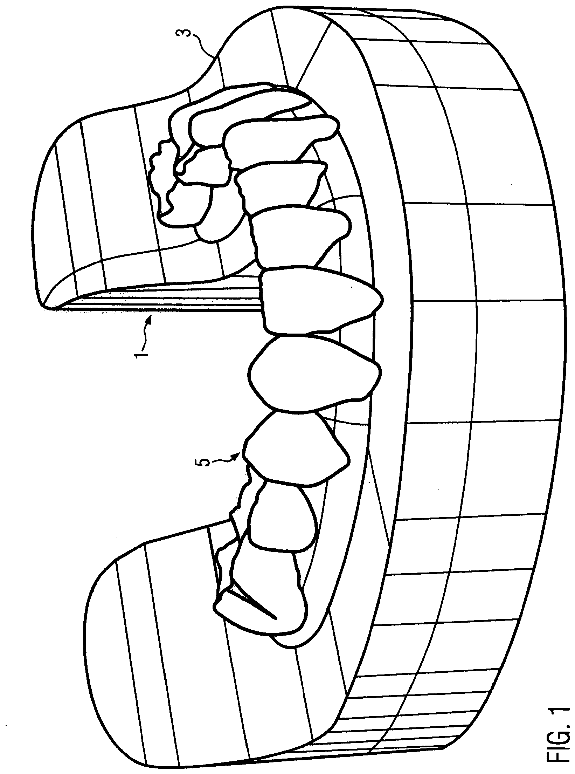

In Figur 1 ist ein Fräsblock 1 dargestellt, der eine Prothesenbasis 3 aufweist, die entsprechend der Kieferform bearbeitet wird, auf der bereits künstlich geformte Zähne 5 eines vollständigen Gebisses vorgesehen sind. Die künstlichen Zähne sind bereits so gearbeitet, dass die Zähne entsprechend einer Okklusionsstellung ausgerichtet sind und somit optimal mit dem nicht dargestellten Oberkiefer zusammenwirken können. In dieser Darstellung sind beispielsweise die Prothesenbasis und die Zähne einstückig ausgebildet, sodass der Herstellungsprozess vereinfacht wurde.

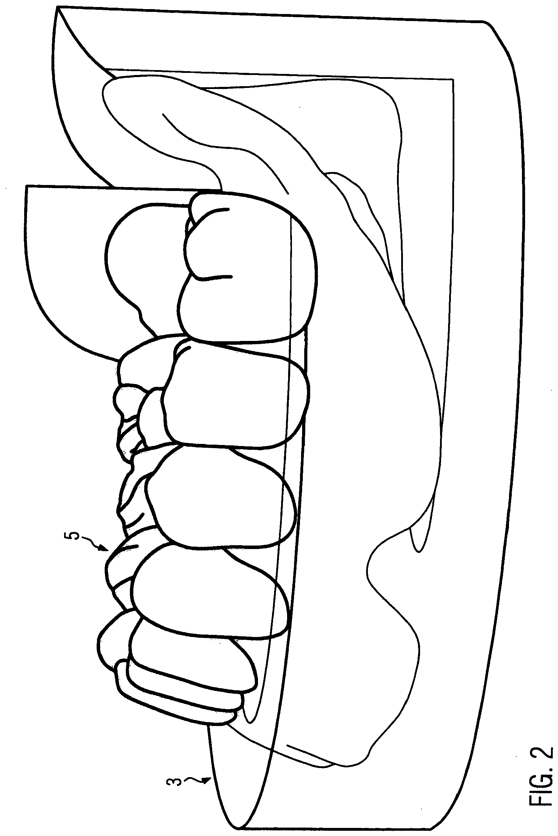

In Figur 2 ist die fertiggestellte Totalprothese des Unterkiefers dargestellt, wobei die Prothesenbasis 3 bereits an die Mundhöhle angepasst ist. Die Figur 2 zeigt deutlich, wie aus der Prothesenbasis die Form der Mundhöhle ausgefräst wird, wobei die Zähne 5, welche in Okklusionsstellung ausgerichtet sind, unbehandelt bleiben. Mit dem anmeldungsgemäßen Verfahren wird somit erreicht, dass lediglich durch Anpassung der Prothesenbasis eine vollständige Totalprothese vorliegt, deren Okklusion stimmig ist und kein weiterer Verfahrensschritt, wie beispielsweise Einpassen in einen Artikulator, erforderlich macht. Auch ein weiterer Verfahrensschritt, wie beispielsweise im Stand der Technik genannt, nämlich die nachträgliche Ausrichtung durch Aufbau der Zähne in Wachs, um die Okklusionsstellung zu erreichen, ist mit dem anmeldungsgemäßen Verfahren nicht erforderlich.

## Patentansprüche

1. Fräsblock (1) zur Herstellung von Teil- oder Totalprothesen, **gekennzeichnet durch** eine entsprechend der Kieferform zu bearbeitenden Prothesenbasis (3), auf der künstlich geformte Zähne (5), vorzugsweise eines vollständigen Gebisses, vorgesehen sind.

2. Fräsblock nach Anspruch 1 , **dadurch gekennzeichnet, dass** die Prothesenbasis und die Zähne einstückig ausgebildet sind.

3. Fräsblock nach Anspruch 1, **dadurch gekennzeichnet, dass** die Prothesenbasis zweiteilig ausgestaltet ist, bestehend aus einem ersten, dem zu bearbeitenden Bereich und aus einem zweiten, dem die künstlich geformten Zähne aufweisenden Bereich, wobei vorzugsweise durch Polymerisation der erste und zweite Bereich zur Prothesenbasis verbunden sind.

4. Fräsblock nach einem der Ansprüche 1 bis 3, **dadurch gekennzeichnet, dass** die Prothesenbasis individuell entsprechend der Kontur der Mundhöhle eines Patienten bearbeitbar ist.

5. Fräsblock nach einem der Ansprüche 1 bis 4, **dadurch gekennzeichnet, dass** als Materialien Keramiken wie Glaskeramik, Leuzitverstärkte Keramik, Aluminiumverstärkte Keramik, Oxidkeramik, Zirkonoxidkeramik, Infiltrationskeramik, Feldspatkeramiken oder Lithium-Disilikat-Keramik verwendet werden.

6. Fräsblock nach einem der Ansprüche 1 bis 4, **dadurch gekennzeichnet, dass** als Materialien PMMA- Kunststoffe, Thermoplasten, Peek Nylon Composite, organisch sowie anorganisch gefüllte Kunststoffe, allergiearme Kunststoffe, ausbrennbare Materialien und/oder gesinterte Materialen verwendet werden.

7. Fräsblock nach einem der Ansprüche 1 bis 4, **dadurch gekennzeichnet, dass** der Fräsblock für den kombinierten Zahnersatz, insbesondere auf Metallbasis bzw. Metallkunststoffbasis bzw. Keramikbasis mit Kunststoffzähnen verwendbar ist.

8. Fräsblocksystem bestehend aus einem ersten Fräsblock für das Obergebiss und einem zweiten Fräsblock für das Untergebiss gemäß einem Fräsblock nach einem der Ansprüche 1 bis 7, wobei jeder Fräsblock eine Prothesenbasis aufweist, die entsprechend der Kontur einer Mundhöhle eines Patienten bearbeitbar ist.

9. Fräsblocksystem nach Anspruch 8, wobei die Zähne des Obergebisses und des Untergebisses zueinander in Okklusionsstellung angeordnet sind.

10. Fräsblocksystem nach einem der Ansprüche 8 oder 9, **dadurch gekennzeichnet, dass** die Zähne entsprechend zur Bissregistrierung ausgerichtet sind.

11. Fräsblocksystem nach einem der Ansprüche 8 bis 10, **dadurch gekennzeichnet, dass** das Fräsblocksystem Teilprothesen zumindest gegenüberliegender Quadranten aufweist.

12. Fräsblocksystem nach einem der Ansprüche 8 bis 11, **dadurch gekennzeichnet, dass** die künstlich geformten Zähne in die Prothesenbasis einpolymerisiert sind.

13. Verfahren zur Herstellung von Teil- oder Totalprothesen, welches die folgenden Schritte aufweist:
a. Herstellen bzw. Bereitstellen eines ersten Fräsblocks für das Obergebiss und eines zweiten Fräsblocks für das Untergebiss, nach einem der Ansprüche 1 bis 12;
b. Bestimmen der Konturdaten der Mundhöhle eines Patienten;
c. Präparieren und/oder Bearbeiten des ersten und zweiten Fräsblocks entsprechend der Konturdaten der Mundhöhle.

14. Verfahren nach Anspruch 13, wobei das Präparieren und/oder Bearbeiten des ersten und zweiten Fräsblocks mittels CAD/CAM Systemen erfolgt.

15. Verfahren nach Anspruch 13 oder 14, wobei die Zähne für die Okklusionsstellung aufgestellt werden.

16. Verfahren nach einem der Ansprüche 13 bis 15, wobei die Zähne nach den Aufstellverfahren Gysi, Gerber, APF, APF NT, TiF, Schreinemaker, Staub Cranial, und/oder weiteren dergleichen Aufstellmethoden aufgestellt werden.

17. Verfahren nach einem der Ansprüche 13 bis 16, wobei die ausgewählten künstlich geformten Zähne auf einen zweiten Bereich der Prothesenbasis aufgestellt werden und dieser zweite Bereich mit einem ersten, dem zu bearbeitenden Bereich der Prothesenbasis zusammengefügt wird.

18. Verfahren nach Anspruch 17, wobei das Zusammenfügen des ersten und zweiten Bereichs durch Polymerisation, durch Kleben, durch Pressen und/oder Verschmelzen erfolgt.

19. Verfahren nach einem der Ansprüche 13 bis 18, wobei die ausgewählten Zähne in der Okklusionsstellung im zweiten Bereich vorpolymerisiert und/oder anschließend mit dem ersten Bereich endpolymerisiert werden.

20. Verfahren nach einem der Ansprüche 13 bis 19, wobei die Prothesenbasis zur vereinfachten Einpassung in die Mundhöhle bzw. Anpassung an die Kieferform des Patienten flexibel ausgestaltet ist.

21. Verfahren nach Anspruch 20, wobei die flexible Prothesenbasis durch Erwärmen, Bestrahlung mit Licht oder durch chemische Reaktion mindestens zweier in der Prothesenbasis vorhandener Komponenten versteift bzw. verfestigt wird.

22. Verfahren nach einem der Ansprüche 13 bis 21, **dadurch gekennzeichnet, dass** die palatinalen und vestibulären Bereiche der Prothesenbasis nicht bearbeitet werden.

## Claims

1. Milling block (1) for producing partial or total prostheses, **characterized by** a prosthesis base (3) which is to be processed according to the shape of the jaw and on which synthetically moulded teeth (5), preferably of a full set of teeth, are provided.

2. Milling block according to Claim 1, **characterized in that** the prosthesis base and the teeth are made in one piece.

3. Milling block according to Claim 1, **characterized in that** the prosthesis base is configured in two parts, consisting of a first region, the region to be processed, and of a second region, the region having the synthetically moulded teeth, the first region and second region preferably being connected to the prosthesis base by polymerization.

4. Milling block according to one of Claims 1 to 3, **characterized in that** the prosthesis base can be processed individually according to the contour of the oral cavity of a patient.

5. Milling block according to one of Claims 1 to 4, **characterized in that** ceramics such as glass ceramic, leucite-reinforced ceramic, aluminium-reinforced ceramic, oxide ceramic, zirconium oxide ceramic, infiltration ceramic, feldspar ceramics or lithium disilicate ceramic are used as materials.

6. Milling block according to one of Claims 1 to 4, **characterized in that** PMMA plastics, thermoplastics, PEEK nylon composite, organically and inorganically filled plastics, low-allergy plastics, out-burnable materials and/or sintered materials are used as materials.

7. Milling block according to one of Claims 1 to 4, **characterized in that** the milling block can be used for the combined dental prosthesis, in particular on a metal base or metal plastic base or ceramic base with plastic teeth.

8. Milling block system consisting of a first milling block for the upper set of teeth and a second milling block for the lower set of teeth in accordance with a milling block according to one of Claims 1 to 7, wherein each milling block has a prosthesis base which can be processed according to the contour of the oral cavity of a patient.

9. Milling block system according to Claim 8, wherein the teeth of the upper set of teeth and of the lower set of teeth are arranged in an occlusion position relative to one another.

10. Milling block system according to either of Claim 8 and 9, **characterized in that** the teeth are oriented according to the bite registration.

11. Milling block system according to one of Claims 8 to 10, **characterized in that** the milling block system has partial prostheses of at least opposite quadrants.

12. Milling block system according to one of Claims 8 to 11, **characterized in that** the synthetically moulded teeth are polymerized into the prosthesis base.

13. Method for producing partial or total prostheses, which method has the following steps:
a. producing or making available a first milling block for the upper set of teeth and a second milling block for the lower set of teeth, according to one of Claims 1 to 12;
b. determining the contour data of the oral cavity of a patient;
c. preparing and/or processing the first and second milling block according to the contour data of the oral cavity.

14. Method according to Claim 13, wherein the preparation and/or processing of the first and second milling block takes place by means of CAD/CAM systems.

15. Method according to Claim 13 or 14, wherein the teeth are set up for the occlusion position.

16. Method according to one of Claims 13 to 15, wherein the teeth are set up according to the Gysi, Gerber, APF, APF NT, TiF, Schreinemaker, Staub Cranial set-up methods and/or further similar set-up methods.

17. Method according to one of Claims 13 to 16, wherein the selected synthetically moulded teeth are set up on a second region of the prosthesis base, and this second region is joined to a first region of the prosthesis base, the region to be processed.

18. Method according to Claim 17, wherein the first and second region are joined together by polymerization, by adhesive bonding, by pressing together and/or melting together.

19. Method according to one of Claims 13 to 18, wherein the selected teeth are pre-polymerized in the occlusion position in the second region and/or then finally polymerized to the first region.

20. Method according to one of Claims 13 to 19, wherein the prosthesis base is flexible in order to permit simplified fitting into the oral cavity and/or adaptation to the shape of the jaw of the patient.

21. Method according to Claim 20, wherein the flexible prosthesis base is stiffened or solidified by heating, by irradiating with light, or by chemical reaction of at least two components present in the prosthesis base.

22. Method according to one of Claims 13 to 21, **characterized in that** the palatal and vestibular regions of the prosthesis base are not processed.

## Revendications

1. Bloc à fraiser (1) pour la fabrication de prothèses partielles ou totales, **caractérisé par** une base de prothèses (3) à usiner en fonction de la forme du maxillaire et sur laquelle des dents moulées artificiellement (5), de préférence une dentition complète, sont prévues.

2. Bloc à fraiser selon la revendication 1, **caractérisé en ce que** la base de prothèse et les dents forment une seule pièce.

3. Bloc à fraiser selon la revendication 1, **caractérisé en ce que** la base de prothèse est réalisée en deux pièces consistant en une première pièce, celle à usiner, et en une seconde pièce, celle présentant les dents moulées artificiellement, les première et seconde pièces étant de préférence raccordées à la base de prothèse par polymérisation.

4. Bloc à fraiser selon une des revendications 1 à 3, **caractérisé en ce que** la base de prothèse peut être usinée individuellement en fonction du contour de la cavité buccale d'un patient.

5. Bloc à fraiser selon une des revendications 1 à 4, **caractérisé en ce qu'**on utilise en tant que matériaux des céramiques comme la céramique de verre, la céramique renforcée à la leucite, la céramique renforcée à l'aluminium, la céramique à l'oxyde, la céramique à l'oxyde de zirconium, la céramique à infiltration, la céramique au feldspath ou la céramique au disilicate de lithium.

6. Bloc à fraiser selon une des revendications 1 à 4, **caractérisé en ce qu'**on utilise en tant que matériaux des plastiques PMMA, des thermoplastiques, du composite de Peek Nylon, des plastiques à charge organique et anorganique, des plastiques faiblement allergènes, des matériaux qui se consument et/ou frittés.

7. Bloc à fraiser selon une des revendications 1 à 4, **caractérisé en ce que** le bloc à fraiser est utilisable pour le remplacement combiné des dents, en particulier sur base métallique ou base plastique et métallique ou sur base céramique avec des dents en plastique.

8. Système de bloc à fraiser composé d'un premier bloc à fraiser pour la dentition supérieure et d'un second bloc à fraiser pour la dentition inférieure conformément à un bloc à fraiser selon une des revendications 1 à 7, dans lequel chaque bloc à fraiser présente une base de prothèse qui peut être usinée en fonction du contour d'une cavité buccale d'un patient.

9. Système de bloc à fraiser selon la revendication 8, dans lequel les dents de la dentition supérieure et de la dentition inférieure sont disposées en position d'occlusion les unes par rapport aux autres.

10. Système de bloc à fraiser selon une des revendications 8 ou 9, **caractérisé en ce que** les dents sont orientées en fonction de l'enregistrement dentaire.

11. Système de bloc à fraiser selon une des revendications 8 à 10, **caractérisé en ce que** le système de bloc à fraiser présente des prothèses partielles de quadrants au moins opposés.

12. Système de bloc à fraiser selon une des revendications 8 à 11, **caractérisé en ce que** les dents moulées artificiellement sont intégrées par polymérisation dans la base de prothèse.

13. Procédé de fabrication de prothèses partielles ou totales comprenant les étapes suivantes:
a. fabrication ou mise à disposition d'un premier bloc à fraiser pour la dentition supérieure et d'un second bloc à fraiser pour la dentition inférieure selon une des revendications 1 à 12 ;
b. définition des données de contour de la cavité buccale d'un patient ;
c. préparation et/ou usinage du premier et du second bloc à fraiser en fonction des données de contour de la cavité buccale.

14. Procédé selon la revendication 13, dans lequel la préparation et/ou l'usinage du premier et du deuxième bloc à fraiser se fait au moyen de systèmes DAO/CAO.

15. Procédé selon la revendication 13 ou 14, dans lequel les dents sont mises en place pour la position d'occlusion.

16. Procédé selon une des revendications 13 à 15, dans lequel les dents sont mises en place d'après les procédés de mise en place Gysi, Gerber, APF, APF NT, TiF, Schreinemaker, Staub, Cranial et/ou d'autres procédés similaires de mise en place.

17. Procédé selon une des revendications 13 à 16, dans lequel les dents artificiellement moulées sélectionnées sont mises en place sur une seconde zone de la base de la prothèse et cette seconde zone est assemblée avec une première zone de la base de prothèse, celle à usiner.

18. Procédé selon la revendication 17, dans lequel l'assemblage des première et seconde zones se fait par polymérisation, par collage, par pressage et/ou par fusion.

19. Procédé selon une des revendications 13 à 18, dans lequel les dents sélectionnées sont pré-polymérisées dans la position d'occlusion dans la seconde zone et/ou subissent ensuite une polymérisation finale avec la première zone.

20. Procédé selon l'une des revendications 13 à 19, dans lequel la base de la prothèse est conçue de manière souple pour s'intégrer de manière simplifiée dans la cavité buccale ou pour s'adapter à la forme du maxillaire du patient.

21. Procédé selon la revendication 20, dans lequel la base souple de la prothèse est rigidifiée ou consolidée par réchauffement, par irradiation à la lumière ou par une réaction chimique d'au moins deux composants présents dans la base de la prothèse.

22. Procédé selon l'une des revendications 13 à 21, **caractérisé en ce que** les zones palatines et vestibulaires de la base de la prothèse ne sont pas usinées.
